# EUROPEAN PATENT SPECIFICATION

(11) **EP 1 504 088 B1**
(45) Date of publication and mention of the grant of the patent: **15.08.2007**
(21) Application number: 03744903.0
(22) Date of filing: 19.03.2003
(51) Int. Cl.: C12N 7/00, A61K 35/76

(54) **BACTERIOPHAGES USEFUL FOR THERAPY AND PROPHYLAXIS OF BACTERIAL INFECTIONS**
ZUR THERAPIE UND PROPHYLAXE BAKTERIELLER INFEKTIONEN GEEIGNETE BAKTERIOPHAGEN
BACTERIOPHAGES UTILES POUR LA THERAPIE ET LA PROPHYLAXIE D'INFECTIONS BACTERIELLES

(30) Priority: 25.03.2002 GB 0207021; 07.08.2002 US 215056
(43) Date of publication of application: 09.02.2005
(73) Proprietor: UNIVERSITY OF WARWICK, Coventry, Warwickshire CV4 7AL (GB)
(72) Inventor: RAPSON, Mark, Edward, Warwickshire CV31 2RJ (GB); BURDEN, Faith, Adelaide, Paignton, Devon TQ3 3DQ (GB); GLANCEY, Liudmilla, Pencheva, Langham, Essex CO4 5PJ (GB); HODGSON, David, Allan, Cheylesmore, Coventry CV3 5HH (GB); MANN, Nicholas, Harold, Leamington Spa, Warwickshire CV32 5UP (GB)
(74) Representative: Elsy, David
(86) International application number: PCT/GB2003/001190
(87) International publication number: WO 2003/080823

(56) References cited:
- WO-A-01/50866
- US-A- 6 121 036
- BRUSSOW HARALD ET AL: "Comparative phage genomics and the evolution of Siphoviridae: Insights from dairy phages." MOLECULAR MICROBIOLOGY, vol. 39, no. 2, January 2001 (2001-01), pages 213-222, XP002245819 ISSN: 0950-382X
- BARROW P A ET AL: "BACTERIOPHAGE THERAPY AND PROPHYLAXIS: REDISCOVERY AND RENEWED ASSESSMENT OF POTENTIAL" TRENDS IN MICROBIOLOGY, ELSEVIER SCIENCE LTD., KIDLINGTON, GB, vol. 5, 1997, pages 268-271, XP000993117 ISSN: 0966-842X
- LEWIN, B.: "Molekularbiologie der Gene" 1998 , SPEKTRUM AKADEMISCHER VERLAG GMBH , HEIDELBERG, BERLIN XP002245820 In particular chapter 13.4. page 319 -page 344

### SEQUENCE LISTING

<110> University of Warwick
<120> Anti-Bacterial Agents
<130> P706370US
<150> GB 0207021.7
   <151> 2002-03-25
<150> US 10/215,056
   <151> 2002-07-08
<160> 16
<170> PatentIn version 3.2
<210> 1
   <211> 20
   <212> DNA
   <213> Artificial
<220>
   <223> PCR primer: FAMILY 1 Forward
<400> 1
   cgtcaactcg gagatatgaa 20
<210> 2
   <211> 20
   <212> DNA
   <213> Artificial
<220>
   <223> PCR primer: FAMILY 1 Reverse
<400> 2
   gtatccgaat ccttcctcgt 20
<210> 3
   <211> 20
   <212> DNA
   <213> Artificial
<220>
   <223> PCR primer: FAMILY 2 Forward
<400> 3
   attcgttgca ctcatgacag 20
<210> 4
   <211> 20
   <212> DNA
   <213> Artificial
<220>
   <223> PCR primer: FAMILY 2 Reverse
<400> 4
   ctcgcaactt ctgctactca
   20
<210> 5
   <211> 20
   <212> DNA
   <213> Artificial
<220>
   <223> PCR primer: FAMILY 3 Forward
<400> 5
   ctgttggcta tgcacgatct
   20
<210> 6
   <211> 20
   <212> DNA
   <213> Artificial
<220>
   <223> PCR primer: FAMILY 3 Reverse
<400> 6
   ctgggaatag gagttaccga
   20
<210> 7
   <211> 20
   <212> DNA
   <213> Artificial
<220>
   <223> PCR primer: FAMILY 4 Forward
<400> 7
   gcaccgtcca catctacatt
   20
<210> 8
   <211> 20
   <212> DNA
   <213> Artificial
<220>
   <223> PCR primer: FAMILY 4 Reverse
<400> 8
   ctgcacgcat gcctgtatat
   20
<210> 9
   <211> 20
   <212> DNA
   <213> Artificial
<220>
   <223> PCR primer: FAMILY 5 Forward
<400> 9
   gcgtgaagct aattctgctg
   20
<210> 10
   <211> 20
   <212> DNA
   <213> Artificial
<220>
   <223> PCR primer: FAMILY 5 Reverse
<400> 10
   actgacacga caacccgtac
   20
<210> 11
   <211> 20
   <212> DNA
   <213> Artificial
<220>
   <223> PCR primer: FAMILY 6 Forward
<400> 11
   gcgaagctat ggctcttgtt
   20
<210> 12
   <211> 20
   <212> DNA
   <213> Artificial
<220>
   <223> PCR primer: FAMILY 6 Reverse
<400> 12
   cacgttgatg tcgttcagtt
   20
<210> 13
   <211> 20
   <212> DNA
   <213> Artificial
<220>
   <223> PCR primer: FAMILY 7 Forward
<400> 13
   gcgaattggt gaagctactg
   20
<210> 14
   <211> 20
   <212> DNA
   <213> Artificial
<220>
   <223> PCR primer: FAMILY 7 Reverse
<400> 14
   agcatgagaa tgccgtaacc
   20
<210> 15
   <211> 20
   <212> DNA
   <213> Artificial
<220>
   <223> PCR primer: FAMILY 8 Forward
<400> 15
   ggcactatca aagagacaac
   20
<210> 16
   <211> 20
   <212> DNA
   <213> Artificial
<220>
   <223> PCR primer: FAMILY 8 Reverse
<400> 16
   ctacatgctc ttgcattgtc
   20

## Description

The application relates to panels of bacteriophages comprising *vir* mutants, compositions containing such mutants, methods of identifying and producing such panels of bacteriophages and to the use of such panels as antibacterial agents, treating bacterial infections and for prophylaxis.

Antibacterial agents, in the form of chemically-based antibiotics (i.e. non-viral agents), such as penicillin or tetracycline, are well known. The problem with such antibiotics is that resistance to them is becoming increasingly common. Mutations conferring antibiotic resistance, or genes encoding antibiotic resistance enzymes, such as penicillinases, are becoming increasingly common in pathogenic bacteria world-wide. Methicillin-resistant *Staphylococcus aureus* (MRSA) bacteria, for example, are an increasingly common form of infection, often acquired during surgery for other causes at hospitals. MRSA infections are extremely difficult to treat using conventional antibiotics.

One alternative approach to treating bacterial infections is to infect the bacteria with a virus, known as a bacteriophage. Such "bacteriophage therapy" was first developed early in the twentieth century, but has been little used in the West since the advent of antibiotics in the 1940s. More extensive work has been carried out in Eastern Europe.

Bacteriophages (also known as "phages") are specific to specific kinds of bacterial cells. They cannot infect the cells of more complex organisms because of major differences in key intracellular machinery, as well as in cell-surface components. Most bacteriophages have structures, such as tails, which enable the bacteriophages to bind to specific molecules on the surface of their target bacteria. Viral DNA within the bacteriophages is then injected through the tail into the host cell, which then directs the production of new bacteriophage.

Different kinds of bacteriophages are found which infect different bacteria. Conventionally, they can be isolated from the environment in which the particular bacterium grows, for example from sewage or faeces. The presence of a bacteriophage may be determined by growing the bacterium in a suitable growth medium, spinning the growth medium to separate off the liquid part of the broth from the bacteria, and passing the separated liquid through a filter with pores small enough to prevent the bacteria getting through the filter. The filtered extract is usually mixed with growth medium, bacteria added, and then spread on, for example, an agar plate. The presence of clear spots, called plaques, on the resulting lawn of bacteria indicates the presence of one or more bacteriophages, which cause the bacteria to lyse.

Bacteriophages which can only kill bacteria are known as "lytic" bacteriophages. Lytic bacteriophages exist outside the bacterial cell in the form of nucleic acid material, often DNA, surrounded by a protein coat. The protein coat usually has one or more molecules attached to it which allow the bacteriophages to attach to specific molecules on the surface of the bacteria. Upon binding to the bacteria the DNA gains entry into the bacterial host where it is transcribed and translated into various proteins necessary for replication and assembly of new bacteriophage. The DNA is also replicated and is packaged into new bacteriophage which are released upon lysis of the bacterial cell.

In addition to lytic bacteriophages there are temperate, or lysogenic bacteriophages. These temperate bacteriophages have two life cycles, one in which they lyse the infected cell, and the other in which they enter the prophage state. Lytic bacteriophages always have to infect from outside, reprogram the host cell and release a burst of bacteriophage through breaking open or lysing. These "lysogenic" bacteriophages may integrate their DNA into the host bacterial DNA leading to a virtually permanent association as a prophage within a specific bacterium and its progeny. Some prophages do not physically integrate into the chromosome, but exist as an autonomous replicon. The prophage directs the synthesis of a repressor which blocks the expression of its own genes and also those of any closely-related lysogenic bacteriophages. Occasionally, the prophage may escape regulation by its repressor. The prophage DNA may then be cut out of the genome by site-specific recombination, replicated, and the progeny released from the host cell, in most cases by lysis.

Lytic bacteriophage have been used to treat bacterial infections. Isolated lytic bacteriophages have been applied to wounds or injected intravenously where they kill bacteria. The advantage of bacteriophages is that they are self-replicating, with as few as one hundred or so bacteriophages being able to kill as many as one hundred million bacteria. The bacteriophages simply replicate themselves by killing bacteria until they have eliminated them from the individual or the environment. WO 01/51066, for example, discloses a method of treating a patient with one or more lytic bacteriophages. Similarly, US 4,957,686 discloses a method of treating dental caries with bacteriophage.

One possible problem with using bacteriophages has been that the patient's own body will often have an immune response against the bacteriophages and eliminate the bacteriophages from blood. US 5,660,812, US 5,688,501 and US 5,766,892 all show methods of selecting bacteriophages to improve the bacteriophage half-life within the blood of a patient to be treated. US 5,811,093 discusses amplifying a gene encoding one of the capsid (coating) proteins (capsid E) so that the bacteriophages survive in an animal's circulatory system for longer. In the case of the latter patent, the modification was identified as a point mutation within a gene.

Conventionally, lysogenic bacteriophages have been considered to be bad candidates for bacteriophage therapy. This is because they can potentially lead to the transfer of genes involved in bacterial pathogenicity. Some bacteriophages carry toxin genes, hence lysogens may exhibit enhanced virulence. However, not all temperate bacteriophages carry virulence genes. Also, bacterial genes can be transferred from one host to another during bacteriophage infection and these genes may include virulence genes. The first process is called bacteriophage conversion and the second is called transduction. Accordingly, use of lysogenic bacteriophages has traditionally been considered to be problematical in that the lysogenic bacteriophages can spread genes responsible for pathogenicity to other bacteria. However, lytic bacteriophages can also cause transduction.

Whilst lytic bacteriophages are known to have been used for treating bacterial infections, identifying lytic bacteriophages is often very time-consuming because lytic bacteriophages for some pathogenic bacteria are relatively rare and hard to isolate.

The inventors have realised that lysogenic bacteriophages appear to be more commonly present in some bacteria. They have realised that by selecting a number of different lysogenic bacteriophages by their ability to infect different strains of bacteria, and, for example, by their lack of genes encoding any toxins or other substances involved in bacterial pathogenicity, they can form a panel of bacteriophages. Members of the panel can be selected, for example to treat different strains of the same bacterium.

The inventors have also recognised that the problem with most lysogenic bacteria is that, upon infecting a cell, the bacteriophage DNA will often integrate into the genome of the bacterium, and not kill the bacterium. This problem is overcome by identifying one or more "*vir*" mutants for use in the panel. Such bacteriophage mutants usually comprise a mutation in an operator region of the temperate bacteriophage DNA which prevents a repressor protein binding to the operator. The repressor protein normally binds to the operator so that the prophage is not transcribed and translated and does not enter the lytic cycle. However, in *vir* mutants the mutation means that the infecting bacteriophage DNA is transcribed and translated and results in the lysis of the bacterium.

The invention provides a pharmaceutical composition and an antibacterial composition as defined in claim 1 or claim 2.

Lysogenic bacteriophages usually comprise DNA encoding one or more genes for integrase, resolvase, transposase, excisionase, *attP,* origin of replication and other genes associated with maintenance of the prophage within the host cell's genome.

A vir mutant, of a temperate bacteriophage, is one that can lytically infect a lysogenic host carrying the wild-type prophage. *vir* mutants contain one or more mutated operator regions in the DNA of the bacteriophage which control the transcription of the prophage DNA, which have been mutated so that they have reduced binding specificities for a repressor protein in comparison with the wild-type prophage. Theoretically a *vir* mutant may arise due to a dominant negative mutation in the repressor protein gene. The repressor protein regulates the operator region and may itself be from the bacteriophage or may be from a different, but related bacteriophage.

The two strains of bacteria may be distinguishable by having different prophages within them. As already indicated, the presence of a prophage within a bacterium will confer protection against infection by the same bacteriophage and may confer protection against different, but related bacteriophages. Accordingly, having a panel of different bacteriophages enables one or more different bacteriophages to be selected so that a panel of two or more bacteriophages may be effective against a range of bacteria, or a single bacteriophage may be selected from the panel in order to treat a specific bacterium.

Preferably, each strain of bacteria is an animal or plant pathogen, especially a human pathogen. Preferably, the bacterium is *Staphylococcus* (especially *S. aureus), Helicobacter* (especially *H. pylori), Klebsiella, Listeria, Mycobacterium, Escherichia* (preferably *E*. *coli,* especially *E. coli 0157),* meningococcus, *Campylobacter, Streptococcus, Enterococcus, Shigella, Pseudomonas, Burkholderia, Clostridium, Legionella, Acinetobacter* or *Salmonella.* Preferably, the panel contains at least 5, at least 10, at least 15, at least 20, at least 25, at least 30, at least 35 or at least 40 different bacteriophages. Preferably, at least 50%, at least 60%, at least 70%, at least 80%, at least 90%, more preferably at least 95%, most preferably 100% of the panel of bacteriophage are *vir* mutants. The panel may also comprise one or more lytic bacteriophages.

The panel may comprise at least one host range change mutant bacteriophage. A host range change mutant is a bacteriophage which, upon isolation from an original host bacterium, is not capable of infecting a second strain of bacterium, but which, upon mutation with a mutating agent, becomes capable of infecting the second strain of bacteria.

Preferably, at least one of the strains of bacteria is resistant to one or more antibiotics. By antibiotics we mean one or more chemical antibiotics. That is, not a functional virus which is capable of infecting a cell. Antibiotics include penicillins, tetracyclines and aminoglycosides. Alternatively the strain of bacteria need not be antibiotic resistant.

Pharmaceutical compositions comprising a panel of bacteriophages, in combination with a pharmaceutically acceptable carrier are provided. Such carriers allow the panel of bacteriophages to be applied to a part of the animal or a patient to be treated, or to be placed within the patient or animal to be treated.

Routes of administration include, but are not limited to orally, nasally, aurally, intravenously, intramuscularly, intraperitoneally, intrathecally, vaginally, rectally, topically, via lumbar punctures, or direct application to the brain or its associated membranes. Administration may be achieved via devices such as aerosols, for use as nasal sprays or inhalers. Further the pharmaceutical composition can be made into tablets or suppositories.

Preferably, the pharmaceutical composition is adapted for topical application, by, for example, mixing the panel or bacteriophage with a suitable cream to allow the bacteriophage to be applied topically to a wound or to the nasal passages. Such creams include paraffin and lanolin-based creams of the types known in the art. Preferably the bacteriophage may be incorporated into bandages or wound dressings.

The bacteriophages may be incorporated into consumables such as soap, hand or face creams, shaving creams and foams, dental floss, tooth powder, toothpaste, etc. This allows the treatment of skin diseases, such as acne, by e.g. washing, or the treatment of dental caries, e.g. by brushing teeth with a toothpaste impregnated with the bacteriophages.

The use of the pharmaceutical compositions of the invention as a medicament or in prophylaxis is also included within the scope of the invention.

The composition may be used as a prophylactic to kill bacteria being carried asymptomatically before they cause a disease. For example, the bacteria may be methicillin-resistant *Staphylococcus aureus* (MRSA) which can cause systemic infections and abscesses. These bacteria may reside in the nasal cavity without causing any apparent disease symptoms. However, the bacteria may be spread from person to person and may cause the disease in people who have a reduced immune capacity. Accordingly, killing the bacteria assists in the prevention of the disease. The panel of bacteriophages may simply be applied in a topical cream via a swab to the nose.

The composition may also be used in combination with a suitable carrier as an antibacterial or antiseptic agent. Such an antibacterial composition may simply be applied to the surface, such as a floor, to be disinfected. Alternatively, utensils, such as surgical utensils, to be disinfected, can simple be placed in a suitable antibacterial solution containing a panel of bacteriophages. The material making up such utensils may also incorporate such bacteriophages.

Further the panel of bacteriophages may be incorporated into materials, such as plastics, by such techniques as bonding, adhesion, or co-polymerisation. The materials may then be used to form equipment such as catheters or cannulars, and the like. Equally, the panel of bacteriophages may be incorporated into such equipment once produced.

Alternatively, a sample of the bacterium to be treated or disinfected may be obtained, the bacterium identified and a bacteriophage suitable for killing that bacterium selected from the panel of bacteriophages.

The bacteriophage may also be used to treat milk and milk products. In an associated function a panel bacteriophage could also be used to prevent or treat mastitis, either by incorporating the panel into the milking cluster, or by using a swab for topical application to the teat.

A further aspect of the invention provides a method of identifying a bacteriophage suitable for killing a bacterium, comprising the steps of:
(i) obtaining a sample of bacterium from a patient, animal, or place infected with a bacterium;
(ii) identifying one or more phage sensitivity characteristics of the bacterium;
(iii) comparing the characteristics from step (ii) with the known host range of each bacteriophage of a library of bacteriophages, the library of bacteriophages comprising a first *vir* mutant bacteriophage capable of infecting and lysing a first strain of bacteria, and a second *vir* mutant capable of infecting and lysing a second strain of bacteria; and
(iv) identifying one or more bacteriophage from the panel suitable for killing the bacterium.

The bacteriophage library may be a collection of bacteriophages containing additional members to the panel of the first aspect of the invention.

One of the characteristics of the bacterium may be the presence or absence of a prophage. The presence of prophage may be determined by polymerase chain reaction (PCR) using a primer pair to amplify part of the prophage DNA from the bacterium.

A further aspect of the invention provides a method of identifying the presence of a prophage in a bacterium providing a pair of primers capable of hybridising under high stringency conditions to a prophage nucleotide sequence, and carrying out PCR to identify the presence of the prophage.

PCR amplifies part of the prophage DNA, which may then be sequenced using conventional sequencing techniques.

Preferably the primers are specific for a prophage-specific sequence such as integrase.

High stringency includes, for example 2 mM MgCl₂ and 48°C annealing temperature.

A still further aspect of the invention provides a machine-readable data storage medium, comprising a data storage material encoded with machine-readable data, wherein the data defines one or more characteristics of each bacteriophage in a panel or library of bacteriophages as defined in the invention. The data storage medium may be a floppy disc, a CD-ROM, a computer memory, or some other machine-readable medium. The data preferably enables the selection of one or more suitable bacteriophage to enable the treatment of a bacterial infection or the disinfection of bacterial contamination.

The invention also provides, within its scope, a method of producing a panel of bacteriophages, according to the first aspect of the invention, comprising isolating a first donor bacterium, identifying one or more temperate bacteriophages, mutating the temperate bacteriophages and isolating one or more *vir* mutants of the bacteriophages.

Preferably, the method of producing the panel comprises the steps of:
(i) isolating the first, donor, bacterium;
(ii) co-culturing the first bacterium with a second, cultivating, strain of bacterium capable of being infected with bacteriophage;
(iii) identifying temperate bacteriophages obtained from the first donor bacterium;
(iv) mutating the temperate bacteriophages identified as step (iii) with a mutagen; and
(v) cultivating the first, donor bacterium with the mutated bacteriophage from step (iv) at the second, to identify *vir* mutants capable of lysing the first, donor bacterium.

Alternatively, instead of step (ii), the induction and isolation of bacteriophages may be brought about via chemical or physical agents.

Host range change mutants may be produced for the panel of bacteriophage by:
(i)isolating a first, donor bacterium of interest;
(ii)co-culturing the first bacterium with a second, cultivating strain of bacterium capable of being infected with bacteriophage;
(iii)identifying one or more temperate bacteriophages obtained from the first, donor bacterium;
(iv)mutating the temperate bacteriophages identified in step (iii) with a mutagen; and
(v)cultivating the mutated temperate bacteriophages obtained from step (iv), a third strain of bacterium previously not infected with the bacteriophage, to identify one or more host range change bacteriophages.

The mutagen used is preferably hydroxylamine. Other mutagens, such as ultraviolet light and other known DNA mutagens may be used.

Alternatively, instead of step (ii), the induction and isolation of bacteriophages may be brought about via chemical or physical agents.

A further problem often found is when a plant or animal, such as a human patient, is found to be suffering from an antibiotic resistant infection. The inventors have realised that it is possible to isolate that bacterial pathogen, identify any lysogenic bacteriophage within that bacterium, and mutate the lysogenic bacteriophage from the bacterial pathogen to produce a vir mutant of the lysogenic bacteriophage. This *vir* mutant may then be admixed with one or more suitable pharmaceutically acceptable carriers, and they may be used to treat the bacterial infection. Accordingly, a bacterial pathogen's own prophages from within its own cells, may be used to kill it.

*vir* mutants identified by such a method may in turn be added to the panel as defined in the first aspect of the invention.

The invention will now be described by way of example only.

### Co-Culture and Isolation of Temperate Staphylophages

An overnight culture containing two *Staphylococcus aureus* isolates (SAI) was incubated at 37°C overnight (o/n), then sterile filtered (0.22µm) after centrifugation in a Hettich Zentrifugen EBA12 centrifuge using a 1116 rotor at 5000 rpm. One of these SAI is termed the cultivating strain and the second, the donor strain. 100 µl of the filtrate was added to 100 µl of o/n culture of cultivating strain, then incubated at room temperature (RT) for 40 min. 3 ml of top agar was added to the co-culture. The mixture was then poured over a Luria Broth (LB) (Sambrook, *et al.* 1989, Molecular Cloning, a Laboratory Approach. 2nd Ed. Cold Spring Harbour Press). + 10 mM Mg²⁺ + 8 mM Ca²⁺ plate and incubated at 37°C o/n. Plaques were picked and incubated o/n at 4°C in PBS + 10 mM Mg²⁺, then streaked onto LB + Mg²⁺ + Ca²⁺ plates. A lawn of the cultivating strain (100 µl cultivating strain o/n + top agar) was poured over the streaked plates. Plaques were picked from the streaked plates and stored on chloroform at 4°C until required.

### Mutagenesis and Mutant Isolation

### Hydroxylamine Mutation

To 0.2 ml bacteria-free filtered bacteriophage stock, 0.4 ml EDTA buffer (0.5 M KPO₄ (pH6.0) + 5 mM EDTA) + 0.2ml sterile water (sH₂O) + 0.1ml sterile 0.2 M MgSO₄ + 0.8 ml hydroxylamine solution was added. The hydroxylamine solution (1 M NH₂OH (pH6.0)) was made fresh by adding 0.56 ml 4 M NaOH to 0.35 g NH₂OH, to a final volume of 5 ml with sH₂O (sterile water). This was incubated at 37°C for 31 hours. A control mixture was made in which the bacteriophage stock was substituted for 0.2 ml sterile sH₂O. After the 31 hours incubation the mixture was added to 8 ml LB + 10 mM MgSO₄ + 8 mM Ca(NO₃)₂ in dialysis tubing. The tubing was placed in 500 ml conical flasks, to which 300 ml LB + Mg²⁺ + Ca²⁺ was added. This was placed at 4°C for 7 hours. The LB + Mg²⁺ + Ca²⁺ was replaced a second and third time. The third after a second 7 hour incubation at 4°C. The bacteriophage stock was placed in a sterile universal bottle and stored at 4°C after a third 7 hours incubation at 4°C in LB + Mg²⁺ Ca²⁺.

### Kill Curve

The 99.9% kill (31 hour) was determined via a time course. Samples were taken at appropriate time points. The samples were diluted in LB + Mg²⁺ + Ca²⁺ and serial diluted. The dilutions were incubated with the cultivating strain at equal volumes of 100 µl at RT for 40 min. To this mixture top agar was added and poured over LB + Mg²⁺ + Ca²⁺ plates. Plaques were counted after the plates were incubated at 37°C o/n. A 10³ fold drop in plaque forming units (pfu) was determined as a 99.9% kill.

### Isolating Virulence (vir) Mutants

Virulence (*vir*) mutants were isolated after expression and enrichment steps. 1 ml of hydroxylamine-treated bacteriophage and 1 ml of o/n cultivating strain were added to 15 ml LB + Mg²⁺ + Ca²⁺ and incubated at 37°C. One hour later 1ml of o/n source strain was added. After overnight incubation, the culture was centrifuged in a Hettich Zentrifugen EBA12 centrifuge using a 1116 rotor at 5000 rpm for 3 min. and sterile filtered (0.22 µm). The filter contains 0.22 µm pores which are too small for bacteria to pass through but are not a barrier to bacteriophage, hence the filtrate is devoid of bacteria but contains the bacteriophage. 100 µl of filtrate was added to 100 µl o/n source strain and incubated at RT for 40 min. To this mixture top agar was added and poured over LB + Mg²⁺ + Ca²⁺ plates. After o/n incubation, a glass pipette was used to pick a single plaque. The plug was then added to an Eppendorf containing 100 µl PBS + Mg²⁺ + Ca²⁺, agitated and incubated o/n at 4°C. Using a sterile loop the stock is streaked onto two LB + Mg²⁺ Ca²⁺ plates. Onto one, a lawn of source strain (3 ml top agar + 100 µl source strain) was poured and onto the second a lawn of cultivating strain (3 ml top agar + 100 µl source strain) was added. These were incubated at 37°C o/n. The bacteriophage was determined a presumptive vir mutant if it was able to infect both the source and cultivating strain. To ensure purity a glass pipette was used to pick a single plaque from the source SAI plate. The plug was then added to an Eppendorf containing 100 µl PBS + Mg²⁺ + Ca²⁺, agitated and incubated o/n at 4°C. This was under-streaked as before, incubated, re-picked, agitated and stored at 4°C.

### vir Controls

To ensure the bacteriophage isolated as a *vir* mutant was not a prophage activated from the cultivating strain controls were implemented. A 15 ml LB + Mg²⁺ + Ca²⁺ aliquot was seeded with the source and cultivating strain. After o/n incubation at 37°C the culture was centrifuged in a Hettich Zentrifugen EBA12 centrifuge using a 1116 rotor at 5000 rpm for 3 min. and sterile filtered (0.22 µm). 100 µl of filtrate was added to 100 µl o/n source strain and incubated at RT for 40 min. To this mixture top agar was added and poured over LB + Mg²⁺ + Ca²⁺ plates. If plaques were seen after o/n incubation at 37°C *vir* mutagenesis was not attempted.

### Isolating hrc (host range change) mutants

*hrc* mutants were isolated after the expression and enrichment steps described above. Some strains were not sensitive to the *vir* mutant bacteriophage: *vir*-resistant host (VRH). 1 ml of hydroxylamine-treated bacteriophage and 1ml of o/n cultivating strain were added to 15 ml LB + Mg²⁺ + Ca²⁺ and incubated at 37°C. One hour later, 1 ml of o/n culture of a VRH strain was added. After overnight incubation the culture was centrifuged in a Hettich Zentrifugen EBA12 centrifuge using a 1116 rotor at 5000 rpm for 3 min. and sterile filtered (0.22 µm). 100 µl of filtrate was added to 100 µl o/n VRH strain and incubated at RT for 40 min. To this mixture top agar was added and poured over LB + Mg²⁺ + Ca²⁺ plates. A glass pipette was used to pick a single plaque. The plug was added to an Eppendorf tube containing 100 µl PBS + Mg²⁺+ Ca²⁺, agitated and incubated o/n at 4°C. Using a sterile loop the stock was streaked onto three LB + Mg²⁺ + Ca²⁺ plates. Onto one, a lawn of source strain (3 ml top agar + 100 µl source strain) was poured and onto the second a lawn of cultivating strain (3 ml top agar + 100 µl source strain) was added. To the third a lawn of the VRH strain was added strain (3 ml top agar + 100 µl VRH strain). These were incubated at 37°C o/n. The bacteriophage was determined a presumptive *hrc* mutant if it was able to infect the VRH strain. It was not expected to infect the source or cultivating strain. To ensure purity a glass pipette was used to pick a single plaque from the source strain plate. The plug was then added to an Eppendorf containing 100 µl PBS + Mg²⁺ + Ca²⁺, agitated and incubated o/n at 4°C. This was under-streaked as before incubated, re-picked, agitated and stored at 4°C.

PBS is described in Sambrook, *et al.*

### hrc Controls

To ensure the bacteriophage isolated as a *hrc* mutant was not a prophage activated from the cultivating or source strain controls were implemented. Two inocula were added to 15 ml LB + Mg²⁺ + Ca²⁺ as follows:

| Strain 'A' | Strain 'B' |
|---|---|
| Source | VRH |
| Cultivating | VRH |

After o/n incubation at 37°C the culture was centrifuged in a Hettich Zentrifugen EBA12 centrifuge using a 1116 rotor at 5000 rpm for 3 min. and sterile filtered (0.22 µm). 100 µl of filtrate was added to 100 µl o/n VRH and incubated at RT for 40 min. To this mixture top agar was added and poured over LB + Mg²⁺ + Ca²⁺ plates. If plaques were seen after o/n incubation at 37°C *hrc* mutagenesis was not attempted.

### Purification of Bacteriophage via Caesium Chloride Gradients

4 ml of stagnant overnight SAI culture was added to four 400 ml aliquots of LB + Mg²⁺ Ca²⁺ in fluted 2 litre conical flasks and agitated (300 rpm) at 37°C. After one hour, 40 µl bacteriophage (2 x 10⁷ pfu) was added to the agitated cultures. After 4 hours the cultures had fully lysed. The cultures were pooled and solid NaCl was added to a final concentration 1M. The lysed culture was centrifuged at 11000 g for 10 min. at 4°C after 1 hour on ice.

10% (w/v) PEG 6000 dissolved in the mixture which was then left to stand overnight on ice at 4°C. The pellet formed after centrifugation at 11000 g for 10 min. at 4°C using a rotor as defined in *Sambrook et al.* (1989) was resuspended in 4 ml PBS. An equal volume of chloroform was added. The mixture was then agitated for 30 sec. The chloroform was removed after centrifugation at 3000 g. Caesium chloride (CsCl) was added to a final concentration of 0.5 g/ml. The suspension was placed on top of a CsCl gradient made in polypropylene tubes containing the following CsCl densities: 1.4, 1.5 and 1.6 g/ml PBS. The tubes were centrifuged at 22,000 rpm (Beckman SW28) for 4 hours. A gauge 21 needle was then inserted carefully through the side of the polypropylene tube to extract the bacteriophage band. Thrice repeated dialysis in PBS + Mg²⁺ + Ca²⁺ for 7 hours removed the CsCl. The purified bacteriophage stock was stored at 4°C until needed.

### Integrase Specific PCR

*Staphylococcus aureus* isolates have been shown to almost always contain at least one temperate bacteriophage integrated into their DNA. Temperate bacteriophages capable of inserting into the host DNA must possess a means of doing so, this is most frequently facilitated by the enzyme, integrase, which catalyses integration of the bacteriophage DNA into the host bacterial DNA.

A number of PCR primers have been designed to amplify and identify integrase genes specifically found within bacteriophage capable of infecting *Staphylococcus aureus.* Such technology could also be directed at similarly important genes in other bacteria/bacteriophage systems, such as the resolvase gene (important in *Listeria* bacteriophage integration). The presence of integrase genes within bacterial DNA would suggest the presence of prophage or phage-related entities such as pathogenicity islands.

At the time of designing the PCR primers there had been approximately 27 *Staphylococcus aureus* bacteriophage integrase genes sequenced. These integrase genes were aligned and their phylogenetic relatedness was determined, by creating a phylogenetic tree. This method showed that there appear to be eight distinct 'Families' of bacteriophage integrase genes found in bacteriophage or bacteriophage-related entities within *Staphylococcus aureus.* The PCR reactions using the aforementioned primers, therefore, enable us to determine what families of bacteriophage or bacteriophage-related elements are present within the *Staphylococcus aureus* DNA.

### PCR Primers

The primers used to amplify and identify the integrase genes of the specific families are as follows:
FAMILY 1 Forward: CGT CAA CTC GGA GAT ATG AA
   FAMILY 1 Reverse: GTA TCC GAA TCC TTC CTC GT
FAMILY 2 Forward: ATT CGT TGC ACT CAT GAC AG
   FAMILY 2 Reverse: CTC GCA ACT TCT GCT ACT CA
FAMILY 3 Forward: CTG TTG GCT ATG CAC GAT CT
   FAMILY 3 Reverse: CTG GGA ATA GGA GTT ACC GA
FAMILY 4 Forward: GCA CCG TCC ACA TCT ACA TT
   FAMILY 4 Reverse: CTG CAC GCA TGC CTG TAT AT
FAMILY 5: Forward: GCG TGA AGC TAA TTC TGC TG
   FAMILY 5 Reverse: ACT GAC ACG ACA ACC CGT AC
FAMILY 6 Forward: GCG AAG CTA TGG CTC TTG TT
   FAMILY 6 Reverse: CAC GTT GAT GTC GTT CAG TT
FAMILY 7 Forward: GCG AAT TGG TGA AGC TAC TG
   FAMILY 7 Reverse: AGC ATG AGA ATG CCG TAA CC
FAMILY 8 Forward: GGC ACT ATC AAA GAG ACA AC
   FAMILY 8 Reverse: CTA CAT GCT CTT GCA TTG TC

### PCR Reaction Conditions

The annealing temperature used for this reaction is 48°C. The PCR reaction is a general reaction with 30 cycles.

A PCR Mastermix for eight reactions is made as follows:
1.5 µl Taq Polymerase - available from Gibco Life Tech
4 µl dNTPs (25 µM) - available from Invitrogen
40 µl 10x Buffer (200 mM Tris.HCl, 500 mM KCI, pH8.4) - available from Gibco Life Tech 16 µl MgCl₂ (50mM)
280 µl Water (sterile, deionized)
3.2 µl Primer mastermix (Forward and Reverse Primer 1:1 ratio) (stock is approximately 10 µM)

45 µl of the above mastermix is then added to 5 µl of the sample to be tested (5 µl of 1 colony suspended in 100 µl water).

The PCR enables the presence of prophages and phage-related entities to be detected and identified in bacteria.

### Volunteer Nasal Experiment Results

The following experiment was designed to assess the efficacy of a topically applied *vir* mutated bacteriophage to control nasal-carriage of MRSA.

### Method:

Ψ134/B1 *vir,* at a concentration of 2x10⁸ pfu / ml, was applied to the anterior nares of a healthy volunteer nasally carrying the source strain, SAI 134. A second volunteer who was MRSA positive, but not a SAI 134 carrier was used as a control. The control volunteer received everything that the non-control volunteer received, except the bacteriophage. Volunteers were both male, 25 years old, of the same weight and stature. Both were final year PhD students working in the same field and living in the same house at the time of the experiment. Both volunteers received the same swab and inoculation protocols.

The PBS-based phage solution was applied to the anterior part of each nostril via cotton-wool buds. The cotton-wool buds were soaked in the PBS-based solution and lightly drained. The solution was applied in three applications each of 20 mins in duration (at times: 0 - 20, 60 - 80 and 120 - 140 mins). A separate cotton-wool buds was introduced to each nostril at each application and the solution was applied via a circular motion, to ensure maximum coverage of the nasal mucosa.

Samples were taken in triplicate and plated out on Baird Parker plates, (which are selective for *S*. *aureus*) at designated time points (0, 20, 60, 80, 120, 140 min, 8, 18, 24 hrs, 2, 3, 4, 5 days). After o/n incubation the plates were observed and the number of colonies present was graded on an arbitrary scale:
0: No colonies
1: <10 colonies
2: 10-100 colonies
3: > 100 colonies

### Results

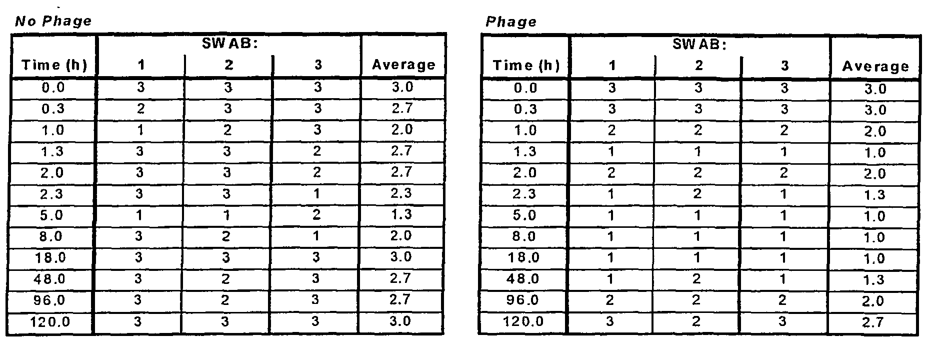

It can be seen that the average number of bacteria reduces quicker in the volunteer that received bacteriophage. The number of bacteria stayed lower for a longer period in this volunteer. By the 5^{th} day the S. *aureus* population has recovered in both volunteers.

### Conclusion

This preliminary experiment suggests that the addition of bacteriophage in the manner described reduces the number of S. *aureus* in the nasal cavity of the volunteer. The level of reduction is above that seen when the adjuvant alone is used. Continued treatment would be necessary to ensure bacterial clearance.

### Animal Wound Model

When Ψ134/B1 *vir* was added to a murine surgical wound model infected with SAI 134, retrieval of bacteria was significantly reduced and the animals were protected from the clinical manifestations of the infection. Animal experiments used 2 x 10⁸ pfu/ml of bacteriophages.

### Production of a Panel of Bacteriophages

The bacteriophage panel will be active against the approximately 100 bacterial isolates from the Warwick University SAI collection, which includes the 17 known EMRSA (epidemic methicillin-resistant *S aureus*) strains and other S. *aureus* isolates from across Europe and publicly available sources of bacteria, such as the NCIMB or the ATCC. Eventually, it is expected that strains of bacteria will be collected from patients at hospitals across the country to get a large collection of strains of bacteria.

Once a temperate bacteriophage is obtained, a virulent (*vir*) mutant is isolated. The wild-type and *vir* mutants are tested against the different strains of bacteria. When at least, for example, 70%, 80%, 90%, 95%, preferably 100% of the strains of bacteria can be controlled by the *vir* mutants, the bacteriophages within the panel may be purified and combined to produce a therapeutic and/or prophylactic product.

The product may be combined with standard pharmacy grade aqueous cream to allow topical application of the product. Alternatively, other pharmaceutically acceptable excipients may be mixed with the panel of bacteriophages.

The panel or bacteriophages may be updated periodically to deal with new strains of resistant bacteria that may emerge.

If a strain of bacterium becomes apparent to which the panel of bacteriophages is ineffective, then the bacteria can be isolated and its prophage obtained. Temperate bacteriophages can be isolated and *vir* mutants produced against the new strain of bacteria, and the *vir* mutants incorporated into the panel of bacteriophages.

In the rare situation that none of the bacteriophages in the bacteriophage panel are able to infect a bacterium associated with an infection, then the bacterium may be isolated and an individual bacteriophage mutated to produce a *vir* mutant to treat that infection.

## Claims

1. A pharmaceutical composition comprising a panel of bacteriophages comprising a first vir mutant bacteriophage capable of infecting and lysing a first strain of bacteria, and a second vir mutant bacteriophage capable of infecting and lysing a second strain of bacteria from the same species of bacteria and wherein the first and second vir mutants have a mutation in the operator region that prevents binding of a repressor protein to an operator, in combination with a pharmaceutically acceptable carrier.

2. An anti-bacterial composition comprising a panel of bacteriophages comprising a first vir mutant bacteriophage capable of infecting and lysing a first strain of bacteria and a second *vir* mutant bacteriophage capable of infecting and lysing a second strain of bacteria from the same species of bacteria and wherein the first and second *vir* mutants have a mutation in the operator region that prevents binding of a repressor protein to an operator, in combination with a carrier.

3. A composition according to claim 1 or claim 2, wherein each said strain of bacteria is a human or animal or plant pathogen.

4. A composition according to claim 1 or claim 2 comprising different bacteriophages effective against different bacteria.

5. A composition according to claim 1 or claim 2 comprising at least one host range change mutant bacteriophage, which upon isolation from an original host bacterium is not capable of infecting a second strain of bacterium, but which upon mutating with a mutagenising agent becomes capable of infecting the second strain of bacterium.

6. A composition according to claim 1 or claim 2 wherein at least one of the said strains of bacteria is resistant to one or more antibiotics.

7. A pharmaceutical composition according to claim 1, adapted for topical application.

8. A pharmaceutical composition according to claim 1 for use as a medicament.

9. A pharmaceutical composition according to claim 1 for the prophylaxis of a bacterial infection.

10. Use of an antibacterial composition according to claim 2 as a disinfectant, with the proviso that the antibacterial composition is not used to treat the human or animal body by surgery or therapy.

11. Use of an antibacterial composition according to claim 2 as an antiseptic, with the proviso that the anti-bacterial composition is not used to treat the human or animal body by surgery or therapy.

12. Use of an antibacterial composition according to claim 2 as a constituent of a surgical or medical device.

13. A method of identifying a bacteriophage suitable for killing a bacterium, comprising the steps of:
(i) identifying one or more phage sensitivity characteristics of a bacterium in a sample obtained from a patient,
(ii) comparing the characteristics from step (ii) with the known host range of each bacteriophage of a library of bacteriophages, said library of bacteriophages comprising a first *vir* mutant bacteriophage capable of infecting and lysing a first strain of bacteria, and a second vir mutant capable of infecting and lysing a second strain of bacteria from the same species of bacteria; and
(iii) identifying one or more bacteriophages from the panel suitable for killing said bacterium.

14. A method according to claim 13, wherein the presence or absence of one or more prophages in said bacterium is determined by PCR using a pair of primers to amplify the prophage DNA from said bacterium.

15. A machine readable data storage medium, comprising a data storage material encoded with machine readable data, wherein the data defines one or more characteristics of each bacteriophage in a panel of bacteriophages in a pharmaceutical or antibacterial composition as defined in claims 1 or 2.

16. A machine readable data storage medium, comprising a data storage material encoded with machine readable data, wherein the data defines one or more characteristics of each bacteriophage in a library of bacteriophages as defined in claim 13.

17. A method of producing a pharmaceutical composition or antibacterial composition according to claim 1 or claim 2, comprising:
(i) cultivating a first donor bacterium;
(ii) identify one or more temperate bacteriophages from said donor bacterium;
(iii) mutating the temperate bacteriophages;
(iv) isolating one or more first *vir* mutants of said bacteriophages, said first *vir* mutant comprising a mutation in an operator region which prevents binding of a repressor to the operator; and
(v) repeating the method steps (i) to (iv) using a second donor bacterium which is a second strain of bacterium from the same species as the first donor bacterium, to identify a second *vir* mutant comprising a mutation in an operator region which prevents binding of a repressor to the operator.

18. A method of producing a composition according to claim 17, wherein:
(i) the first donor bacterium is co-cultured with a second, cultivating strain of bacterium infected with bacteriophage; and
(ii) said one or more temperate bacteriophages are identified.

19. A method of producing a composition according to claim 17, wherein:
(i) the donor bacterium is induced to produce temperate bacteriophages with a chemical or physical agent; and
(ii) said one or more temperate bacteriophage are identified.

20. A method of producing a pharmaceutical or antibacterial composition according to claim 5, comprising:
(i) isolating a first, donor, bacterium of interest;
(ii) either (a) co-culturing the first bacterium with a second, cultivating strain of bacterium infected with bacteriophage or (b) inducing said donor bacterium to produce temperate bacteriophage with a chemical or physical agent;
(iii) identifying one or more temperate bacteriophages obtained from said first, donor, bacterium;
(iv) mutating said temperate bacteriophages identified in step (iii) with a mutagen; and
(v) cultivating the mutated temperate bacteriophages obtained from step (iv), with a third strain of bacterium previously not infected with the bacteriophage, to identify one or more host range change bacteriophages.

## Patentansprüche

1. Pharmazeutische Zusammensetzung, die in Kombination mit einem pharmazeutisch zulässigen Träger ein Feld von Bakteriophagen mit einem ersten "vir"-mutierten Bakteriophagen, der einen ersten Bakterienstamm infizieren und auflösen kann, und einem zweiten "vir"-mutierten Bakteriophagen, der einen zweiten Bakterienstamm von der gleichen Art von Bakterien infizieren und auflösen kann, wobei der erste und der zweite "vir"-Mutant eine Mutation im Operatorbereich haben, die eine Bindung eines Repressorproteins an einen Operator verhindert, aufweist.

2. Antibakterielle Zusammensetzung, die in Kombination mit einem Träger ein Feld von Bakteriophagen mit einem ersten "vir"-mutierten Bakteriophagen, der einen ersten Bakterienstamm infizieren und auflösen kann, und einem zweiten "vir"-mutierten Bakteriophagen, der einen zweiten Bakterienstamm von der gleichen Art von Bakterien infizieren und auflösen kann, wobei der erste und der zweite "vir"-Mutant eine Mutation im Operatorbereich haben, die eine Bindung eines Repressorproteins an einen Operator verhindert, aufweist.

3. Zusammensetzung nach Anspruch 1 oder 2, bei welcher jeder der Bakterienstämme ein menschlicher oder tierischer oder pflanzlicher Krankheitserreger ist.

4. Zusammensetzung nach Anspruch 1 oder 2, mit unterschiedlichen Bakteriophagen, die gegen unterschiedliche Bakterien wirksam sind.

5. Zusammensetzung nach Anspruch 1 oder 2, mit wenigstens einem wirtsbereichsveränderungs-mutierten Bakteriophagen, der bei einer Isolierung aus einer ursprünglichen Wirtsbakterie einen zweiten Bakterienstamm nicht infizieren kann, aber der beim Mutieren mit einem Mutationsmittel in die Lage versetzt wird, den zweiten Bakterienstamm zu infizieren.

6. Zusammensetzung nach Anspruch 1 oder 2, bei welcher wenigstens einer der Bakterienstämme gegen eine oder mehrere Antibiotika resistent ist.

7. Pharmazeutische Zusammensetzung nach Anspruch 1, geeignet zur lokalen Anwendung.

8. Pharmazeutische Zusammensetzung nach Anspruch 1 zur Verwendung als Medikament.

9. Pharmazeutische Zusammensetzung nach Anspruch 1 für die Prophylaxe einer bakteriellen Infektion.

10. Verwendung einer antibakteriellen Zusammensetzung nach Anspruch 2 als ein Desinfektionsmittel, unter dem Vorbehalt, dass die antibakterielle Zusammensetzung nicht verwendet wird, um den menschlichen oder tierischen Körper chirurgisch oder therapeutisch zu behandeln.

11. Verwendung einer antibakteriellen Zusammensetzung nach Anspruch 2 als ein Antiseptikum, unter dem Vorbehalt, dass die antibakterielle Zusammensetzung nicht verwendet wird, um den menschlichen oder tierischen Körper chirurgisch oder therapeutisch zu behandeln.

12. Verwendung einer antibakteriellen Zusammensetzung nach Anspruch 2 als ein Bestandteil einer chirurgischen oder medizinischen Vorrichtung.

13. Verfahren zum Identifizieren eines Bakteriophagen, der eine Bakterie töten kann, mit den Schritten:
(i) Identifizieren einer oder mehrerer Phagenempfindlichkeitseigenschaften einer Bakterie in einer von einem Patienten erhaltenen Probe;
(ii) Vergleichen der Eigenschaften aus Schritt (i) mit dem bekannten Wirtsbereich jedes Bakteriophagen einer Sammlung von Bakteriophagen, wobei die Sammlung von Bakteriophagen einen ersten "vir"-mutierten Bakteriophagen, der einen ersten Bakterienstamm infizieren und auflösen kann, und einen zweiten "vir"-mutierten Bakteriophagen, der einen zweiten Bakterienstamm von der gleichen Art von Bakterien infizieren und auflösen kann, aufweist; und
(iii) Identifizieren eines oder mehrerer Bakteriophagen aus dem Feld, die zum Töten der Bakterie geeignet sind.

14. Verfahren nach Anspruch 13, bei welchem das Vorhandensein oder die Abwesenheit eines oder mehrerer Prophagen in der Bakterie durch PCR unter Verwendung eines Paares von Primern zum Verstärken der Prophagen-DNA aus der Bakterie bestimmt wird.

15. Maschinenlesbares Datenspeichermedium mit einem Datenspeichermaterial, das mit maschinenlesbaren Daten codiert ist, wobei die Daten eine oder mehrere Eigenschaften jedes Bakteriophagen in einem Feld von Bakteriophagen in einer pharmazeutischen oder antibakteriellen Zusammensetzung, wie sie in Anspruch 1 oder 2 definiert ist, definieren.

16. Maschinenlesbares Datenspeichermedium mit einem Datenspeichermaterial, das mit maschinenlesbaren Daten codiert ist, wobei die Daten eine oder mehrere Eigenschaften jedes Bakteriophagen in einer Sammlung von Bakteriophagen, wie sie in Anspruch 13 definiert ist, definiert.

17. Verfahren zum Herstellen einer pharmazeutischen Zusammensetzung oder antibakteriellen Zusammensetzung nach Anspruch 1 oder 2, mit
(i) Kultivieren einer ersten Donorbakterie;
(ii) Identifizieren eines oder mehrerer gemäßigter Bakteriophagen aus der Donorbakterie;
(iii) Mutieren der gemäßigten Bakteriophagen;
(iv) Isolieren eines oder mehrerer erster "vir"-Mutanten der Bakteriophagen, wobei der erste "vir"-Mutant eine Mutation in einem Operatorbereich aufweist, die eine Bindung eines Repressors an den Operator verhindert; und
(v) Wiederholen der Verfahrensschritte (i) bis (iv) unter Verwendung einer zweiten Donorbakterie, die ein zweiter Bakterienstamm von der gleichen Art wie die erste Donorbakterie ist, um einen zweiten "vir"-Mutanten mit einer Mutation in einem Operatorbereich, der eine Bindung eines Repressors an den Operator verhindert, zu identifizieren.

18. Verfahren zum Herstellen einer Zusammensetzung nach Anspruch 17, bei welchem
(i) die erste Donorbakterie gemeinsam mit einem zweiten kultivierten Bakterienstamm, der mit dem Bakteriophagen infiziert ist, kultiviert wird; und
(ii) die einen oder mehreren gemäßigten Bakteriophagen identifiziert werden.

19. Verfahren zum Herstellen einer Zusammensetzung nach Anspruch 17, bei welchem
(i) die Donorbakterie mit einem chemischen oder physikalischen Mittel stimuliert wird, um gemäßigte Bakteriophagen zu erzeugen; und
(ii) die einen oder mehreren gemäßigten Bakteriophagen identifiziert werden.

20. Verfahren zum Herstellen einer pharmazeutischen oder antibakteriellen Zusammensetzung gemäß Anspruch 5, mit
(i) Isolieren einer ersten Donorbakterie von Interesse;
(ii) entweder (a) Kultivieren der ersten Bakterie gemeinsam mit einem zweiten kultivierten Bakterienstamm, der mit einem Bakteriophagen infiziert ist, oder (b) Stimulieren der Donorbakterie mit einem chemischen oder physikalischen Mittel, um einen gemäßigten Bakteriophagen zu erzeugen;
(iii) Identifizieren eines oder mehrerer gemäßigter Bakteriophagen, die man aus der ersten Donorbakterie erhält;
(iv) Mutieren der in Schritt (iii) identifizierten gemäßigten Bakteriophagen mit einem Mutagen; und
(v) Kultivieren der aus Schritt (iv) erhaltenen mutierten gemäßigten Bakteriophagen mit einem dritten Bakterienstamm, der zuvor nicht mit dem Bakteriophagen infiziert wurde, um einen oder mehrere Wirtsbereichsveränderungs-Bakteriophagen zu identifizieren.

## Revendications

1. Composition pharmaceutique comprenant un panel de bactériophages comprenant un premier bactériophage mutant vir capable d'infecter et de lyser une première souche de bactéries, et un second bactériophage mutant vir capable d'infecter et de lyser une seconde souche de bactéries de la même espèce de bactérie et dans laquelle le premier et le second mutants vir ont une mutation dans la région d'opérateur qui empêche la liaison d'une protéine réprésseur à un opérateur, en combinaison avec un porteur acceptable du point de vue pharmaceutique.

2. Composition anti-bactérienne comprenant un panel de bactériophages comprenant un premier bactériophage mutant vir capable d'infecter et de lyser une première souche de bactéries et un second bactériophage mutant *vir* capable d'infecter et de lyser une seconde souche de bactéries de la même espèce de bactéries et dans laquelle le premier et le second mutants vir ont une mutation dans la région d'opérateur qui empêche la liaison d'une protéine réprésseur à un opérateur, en combinaison avec un porteur.

3. Composition selon la revendication 1 ou la revendication 2, dans laquelle chacune des dites souches de bactéries est un germe pathogène humain, animal ou végétal.

4. Composition selon la revendication 1 ou la revendication 2 comprenant différents bactériophages efficaces contre différentes bactéries.

5. Composition selon la revendication 1 ou la revendication 2 comprenant au moins un bactériophage mutant à changement de plage hôte qui en cas d'isolation par rapport à une bactérie hôte d'origine n'est pas capable d'infecter une seconde souche de bactéries, mais qui en cas de mutation avec un agent mutagène devient capable d'infecter la seconde souche de bactéries.

6. Composition selon la revendication 1 ou la revendication 2 dans laquelle au moins une desdites souches de bactéries est résistante à un ou plusieurs antibiotiques.

7. Composition pharmaceutique selon la revendication 1, adaptée pour une application directe.

8. Composition pharmaceutique selon la revendication 1 pour être utilisée comme médicament.

9. Composition pharmaceutique selon la revendication 1 pour la prophylaxie d'une infection bactérienne.

10. Utilisation d'une composition antibactérienne selon la revendication 2 comme désinfectant, avec la condition que la composition antibactérienne n'est pas utilisée pour traiter le corps humain ou animal par chirurgie ou thérapie.

11. Utilisation d'une composition antibactérienne selon la revendication 2 comme antiseptique, avec la condition que la composition antibactérienne n'est pas utilisée pour traiter le corps humain ou animal par chirurgie ou thérapie.

12. Utilisation d'une composition antibactérienne selon la revendication 2 comme constituant d'un dispositif chirurgical ou médical.

13. Procédé d'identification d'un bactériophage adapté pour tuer une bactérie, comprenant les étapes consistant à :
(i) identifier une ou plusieurs caractéristiques de sensibilité au phage d'une bactérie dans un échantillon obtenu sur un patient
(ii) comparer les caractéristiques de l'étape (ii) à la plage hôte connue de chaque bactériophage d'une bibliothèque de bactériophages, ladite bibliothèque de bactériophages comprenant un premier bactériophage mutant *vir* capable d'infecter et de lyser une première souche de bactéries et un second mutant *vir* capable d'infecter et de lyser une seconde souche de bactéries de la même espèce de bactéries ; et
(iii) identifier un ou plusieurs bactériophages dans le panel adapté pour tuer ladite bactérie.

14. Procédé selon la revendication 13, dans lequel la présence ou l'absence d'un ou de plusieurs prophages dans ladite bactérie est déterminée par PCR en utilisant une paire d'amorces pour amplifier le DNA du prophage de ladite bactérie.

15. Moyen de stockage des données lisibles par une machine, comprenant un matériau de stockage de données encodé avec des données lisibles en machine, dans lequel les données définissent une ou plusieurs caractéristiques de chaque bactériophage dans un panel de bactériophages dans une composition pharmaceutique ou antibactérienne telle que définie dans les revendications 1 ou 2.

16. Moyen de stockage des données lisibles par une machine, comprenant un matériau de stockage de données encodé avec des données lisibles en machine, dans lequel les données définissent une ou plusieurs caractéristiques de chaque bactériophage dans une bibliothèque de bactériophages telle que définie dans la revendication 13.

17. Procédé de production d'une composition pharmaceutique ou d'une composition antibactérienne selon la revendication 1 ou la revendication 2, comprenant :
(i) la culture d'une première bactérie donneuse ;
(ii) l'identification d'un ou de plusieurs bactériophages tempérés de ladite bactérie donneuse ;
(iii) la mutation des bactériophages tempérés ;
(iv) l'isolation d'un ou de plusieurs mutants *vir* desdits bactériophages, ledit premier mutant *vir* comprenant une mutation dans une région d'opérateur qui empêche la liaison d'un répresseur à l'opérateur ; et
(v) la répétition des étapes du procédé (i) à (iv), en utilisant une seconde bactérie donneuse qui est une seconde souche de bactérie de la même espèce que la première bactérie donneuse, pour identifier un second mutant vir comprenant une mutation dans une région d'opérateur qui empêche la liaison d'un répresseur à l'opérateur.

18. Procédé de production d'une composition selon la revendication 17, dans lequel :
(i) la première bactérie donneuse est co-cultivée avec une seconde souche de culture de bactérie infectée de bactériophages ; et
(ii) lesdits bactériophages au nombre de une ou plusieurs sont identifiées.

19. Procédé de production d'une composition selon la revendication 17, dans lequel :
(i) la bactérie donneuse est conduite à produire des bactériophages tempérés à l'aide d'un agent chimique ou physique ; et
(ii) ledit bactériophage au nombre de un ou plusieurs est identifié.

20. Procédé de production d'une composition pharmaceutique ou antibactérienne selon la revendication 5, comprenant les étapes consistant à :
(i) isoler une première bactérie donneuse intéressante ;
(ii) soit (a) co-cultiver la première bactérie avec une seconde souche de bactéries infectée par un bactériophage ou (b) induire ladite bactérie donneuse à produire un bactériophage tempéré avec un agent chimique ou physique ;
(iii) identifier un ou plusieurs bactériophages tempérés obtenus à partir de ladite première bactérie donneuse ;
(iv) faire muter lesdits bactériophages tempérés identifiés à l'étape (iii) avec un mutagène ; et
(v) cultiver les bactériophages tempérés mutés obtenus à partir de l'étape (iv), avec une troisième souche de bactéries auparavant non infectées avec le bactériophage, pour identifier un ou plusieurs bactériophages à changement de plage hôte.
